# EUROPÄISCHE PATENTSCHRIFT

(11) **EP 3 053 553 B1**
(45) Veröffentlichungstag und Bekanntmachung des Hinweises auf die Patenterteilung: **18.09.2019**
(21) Anmeldenummer: 15000369.7
(22) Anmeldetag: 09.02.2015
(51) Int. Cl.: A61F 9/007, A61F 9/00

(54) **Vorrichtung zur Prävention von Achsenmyopie des Auges**
Device for prevention of axial myopia of the eye
Dispositif de prévention de la myopie axiale

(43) Veröffentlichungstag der Anmeldung: 10.08.2016
(73) Patentinhaber: IROC Services AG, 6300 Zug (CH)
(72) Erfinder: Prof. Dr. Dr. Seiler, Theo, 8002 Zürich (CH); Seiler, Theo, jun., 8002 Zürich (CH); Seiler, Stefan, 8002 Zürich (CH)
(74) Vertreter: Frenkel, Matthias Alexander

(56) Entgegenhaltungen:
- EP-A1- 1 854 438
- WO-A2-2014/145666
- IT-B- 1 227 189
- US-A- 4 549 529
- US-A1- 2012 209 051

## Beschreibung

Die Erfindung betrifft eine Vorrichtung zur Vorbeugung und/oder Behandlung von Achsenmyopie des Auges mit zumindest einem Band, das eingerichtet ist, von außen auf die Sklera des Auges aufgelegt oder benachbart der Sklera angeordnet zu werden, einer oder mehreren Strahlungsquellen zur Erzeugung von elektromagnetischer Strahlung, die im Band erzeugbar ist oder in das Band einkoppelbar ist, um aus dem Band in die Sklera gestrahlt zu werden.

Eine solche Vorrichtung ist aus der WO 2007/128581 A2 bekannt.

Bei der sogenannten Myopie oder Kurzsichtigkeit können weit entfernte Objekte schlechter gesehen werden als nahegelegene. Die Myopie bezeichnet eine Form der Fehlsichtigkeit, bei der der Fokus vor der Netzhaut liegt. Dabei werden zwei prinzipielle Formen der Kurzsichtigkeit unterschieden, zum einen die "Brechungsmyopie" bei normaler Augenachsenlänge, aber zu starker Brechkraft, und die sogenannte "Achsenmyopie" bei normaler Brechkraft, aber zu langer Augenachse. Für die erste Form, die "Brechungsmyopie", kennt der Stand der Technik vielfältige ophthalmologische Vorrichtungen, die der Korrektur dieses Sehfehlers dienen.

Die zweite Form - die "Achsenmyopie" - beschreibt eine häufig auftretende Erkrankung des Auges, bei der das Auge durch (passives) Sklerawachstum länger wird. Dies erfolgt insbesondere in den ersten drei Lebensjahrzehnten. Hierbei kommt es zu einer allmählichen und übermäßigen Verlängerung des Augapfels. Die Ursachen hierfür sind noch nicht gefunden. Es wird angenommen, dass die genetische Veranlagung eine wichtige Rolle spielt.

Derzeit sind drei Formen der "Achsenmyopie" bekannt: die Schulmyopie, die gutartige progressive Myopie und die bösartige progressive Myopie.

Bei der Schulmyopie beginnt der Augapfel sich kurz vor der Pubertät (im Alter zwischen 10 und 12 Jahren) zu strecken und verstärkt die Kurzsichtigkeit kontinuierlich, bis das Körperwachstum mit etwa 20-25 Jahren abgeschlossen ist. Dabei können die Augen z.B. eine Brechkraft von bis zu minus 4 Dioptrien erreichen.

Bei der gutartigen progressiven Myopie stabilisiert sich die Brechkraft erst mit 30 Jahren. Dann kann die Kurzsichtigkeit bei bis zu 12 Dioptrien liegen.

Schließlich schreitet bei der bösartigen progressiven Myopie die unregelmäßige Dehnung des Augapfels auch bis ins höhere Alter weiter fort. Dann beginnt der Glaskörper zunehmend an der Netzhaut zu zerren, weil diese nicht mit der Lederhaut (Sklera) mitwächst. Hierdurch steigt das Risiko einer Netzhautablösung.

In der Ophthalmologie ist seit mehr als 10 Jahren bekannt, mittels eines sogenannten Photosensibilisators und elektromagnetischer Strahlung die biomechanischen und biochemischen Eigenschaften von Augengewebe, insbesondere der Kornea und Sklera, zu therapeutischen Zwecken zu ändern.

Der menschliche Augapfel wird durch die äußere Augenhaut begrenzt. Durch den Augeninnendruck wird die kollagenhaltige äußere Augenhaut gespannt und verleiht dem gesunden Augapfel eine annähernd sphärische Form. Im mittleren und hinteren Augapfelbereich besteht die äußere Augenhaut aus der weißen Lederhaut (Sklera). Im vorderen Bereich befindet sich die für sichtbares Licht durchlässige Hornhaut (Kornea).

Eine bekannte Therapie stabilisiert die Augenhaut durch Vernetzung (sog. "crosslinking"). Die Behandlung erlaubt eine photochemische, nichtgewebeabtragende Stabilisierung oder Veränderung der biomechanischen und biochemischen Eigenschaften der Kornea. Eine Photosensibilisatorlösung wird in das zu verändernde Augengewebe eingebracht und einer Primärstrahlung ausgesetzt. Als Primärstrahlung wird elektromagnetische Strahlung im Wellenlängenbereich von etwa z.B. 300 nm bis 800 nm (UV-A-Strahlung oder sichtbares Licht) eingesetzt.

Entsprechende Vorrichtungen zur Behandlung der äußeren Augenhaut sind aus den Druckschriften WO 2007/128581 A2 und WO 2008/000478 A1 bekannt.

Die EP 1 561 440 B1 beschreibt eine Vorrichtung, bei der mit einem relativ komplexen Aufbau eine homogene Verteilung der Strahlung im okulärem Gewebe erzeugt wird. Ein Formkörper wird dort auf die Hornhaut aufgesetzt, um diese in eine gewünschte Form zu bringen während mittels elektromagnetischer Strahlung und des Photosensibilisators das okuläre Gewebe hinsichtlich seiner Festigkeit geändert wird.

Eine Vorrichtung gemäß WO 2007/128581 A2 dient der Verfestigung der sich im hinteren Augenabschnitt befindenden Sklera. Die Primärstrahlung kann dabei durch das Innere des Auges oder durch von außen aufliegenden Kissen auf die Sklera einwirken. Durch einen Photomediator oder Photosensibilisator wird eine Vernetzung in der Sklera bewirkt. Dadurch wird einem Sklerawachstum entgegengewirkt und ein Fortschreiten der Achsenmyopie unterbunden.

Die EP 1 854 438 A1 beschreibt eine ophthalmalogische Vorrichtung zur Vorbeugung einer Myopie, bei der die Sklera mittels eines Photosensibilisators verfestigt wird.

Die WO 2008/000478 A1 beschreibt ein Bestrahlungssystem zur biomechanischen Stabilisierung der Kornea. Auch hier kann in Verbindung mit einem Photosensibilisator eine Vernetzung an der Kornea bewirkt werden. Das Bestrahlungssystem bietet die Möglichkeit spezifische Erkrankungen, wie den Keratokonus zu therapieren.

Das Dokument WO 2014/145666 A2 offenbart einen äquatorialen Einsatz, der dazu ausgestaltet ist, um den Äquator des Auges gelegt zu werden, und Lichtleiter aufweist, um Licht im Nahinfraroten, Sichtbaren oder UVA-Bereich von einer Lichtquelle zu empfangen und dem Gewebe der Sklera zuzuführen. Ferner beschreibt dieses Dokument vierbändige Einsätze, die dazu ausgebildet sind, auf die hintere Sklera gelegt zu werden, und Abmessungen von etwa 80 mm x 8 mm x 0,5 mm aufweisen. Jeder vierbändige Einsatz enthält eine sichtbare mikro-LED, die vom Operateur von der Vorderseite gesehen werden kann, um die ordnungsgemäße Positionierung des vierbändigen Einsatzes zu erleichtern.

Im Dokument US 2012/0209051 A1 ist ein äquatoriales Sklera-Mattensystem mit einer äquatorialen Sklera-Matte beschrieben, welches Komponenten für die Zuführung von Riboflavin und für die Zuführung von UV-Strahlung an Skleragewebe aufweist. Riboflavin wird von einem Riboflavinbehälter in die äquatoriale Sklera-Matte gepumpt, wonach dieses mit der Oberfläche der Sklera in Kontakt gelangt und absorbiert wird. Im Anschluss an diese Absorption wird das Riboflavin mittels UV-LEDs aktiviert, die auf der inneren konkaven Oberfläche der äquatorialen Sklera-Matte angeordnet sind.

Die Änderung der Gestalt und/oder von mechanischen Eigenschaften von Augengewebe, insbesondere der Hornhaut und allgemein der Sklera mittels eines eingebrachten Photosensibilisators und elektromagnetischer Strahlung ist als solche im Stand der Technik, insbesondere wie oben genannt, gut bekannt. Hinsichtlich der chemischen Zusammensetzung des Photosensibilisators wird auf den Stand der Technik verwiesen, auch hinsichtlich der eingesetzten Art der elektromagnetischen Strahlung, insbesondere der geeigneten Wellenlängen in Verbindung mit bestimmten Photosensibilisatoren.

Als Photosensibilisator (auch Photomediator genannt) haben sich zur Zeit insbesondere Riboflavin und Bengalrosa sowie weitere Substanzen bewährt.

Der Erfindung liegt die Aufgabe zugrunde, eine Vorrichtung zur Vorbeugung und/oder Behandlung bzw. zur Verhinderung eines Fortschreitens von Achsenmyopie des Auges bereitzustellen, mit der gegenüber dem Stand der Technik verbesserte Ergebnisse erreichbar sind.

Hierzu stellt die Erfindung eine Vorrichtung gemäß Anspruch 1 bereit.

Die erfindungsgemäße Vorrichtung wird bevorzugt eingesetzt zur Behandlung einer Sklera, in die ein sogenannter Photomediator eingebracht ist, wie zum Beispiel Riboflavin oder Bengalrosa. Mittels der Vorrichtung wird dann elektromagnetische Strahlung zur Aktivierung des Photosensibilisators in die Sklera eingestrahlt (vgl. den oben zitierten Stand der Technik) und es erfolgt so eine Änderung der biomechanischen Eigenschaften der Sklera dahingehend, dass einer Längendehnung (in Richtung der optischen Achse des Auges) entgegengewirkt wird. Die Erfindung betrifft also insbesondere die Prävention von Achsenmyopie.

Ein beispielhaftes Verfahren zur Vorbeugung von Achsenmyopie sieht folgende Schritte vor:
Eröffnen der Bindehaut des Auges, Anschlingen der geraden Augenmuskeln, stumpfe Freipräparation von Muskeln und Sklera (diese Schritte sind ähnlich dem operativen Vorgehen bei der sogenannten Cerclage, einer wohlbekannten Operationstechnik bei Netzhautablösung), Spülen der Sklera mit dem Photomediator für eine vorgegebene Zeitspanne (von zum Beispiel 20 min), Anlegen des Bandes entsprechend der erfindungsgemäßen Vorrichtung auf Höhe des Äquators des Auges in der Art eines den Augapfel umfassenden Gürtels (unter den Muskeln), Einstrahlen elektromagnetischer Energie mittels des Bandes radial nach innen in die Sklera (radial in Bezug auf die optische Achse) für eine vorgegebene Zeitspanne von zum Beispiel 15 min derart, dass die Strahlung insbesondere in den vom gürtelartigen Band umfassten Bereich der Sklera einwirkt zur Aktivierung des Photomediators, Entfernen des Bandes, Spülen der Sklera mit einer geeigneten Spüllösung (z.B. BSS), Entfernen der Muskelschlingen, Verschluss der Bindehaut.

Das erfindungsgemäße Band besteht bevorzugt aus einem Gewebe, das entweder im Wesentlichen oder ausschließlich aus Lichtleitfasern besteht oder aus einer Mischung von Textilfasern und Lichtleitfasern (Glasfasern).

Das zum Gürtel für den Augapfel formbare Band hat gemäß einer bevorzugten Ausgestaltung eine Breite in Richtung der optischen Achse von 3 bis 12 mm, weiter bevorzugt im Bereich von 4 bis 10 mm, und besonders bevorzugt im Bereich von 5 bis 8 mm.

Das Band und die elektromagnetische Strahlung werden bevorzugt so eingestellt, dass die Sklera homogen bestrahlt wird, d.h. die Strahlungsenergie pro Flächeneinheit ist im gesamten Bereich der bestrahlten Sklera im Wesentlichen gleich.

Die Strahlungserzeugung kann auf verschiedene Weisen erfolgen:
Zum einen ist es möglich, eine Strahlungsquelle vorzusehen, die extern in Bezug auf das Band angeordnet ist derart, dass die von der Strahlungsquelle erzeugte Strahlung über zum Beispiel einen oder mehrere Lichtleiter zum Band geführt und dort in das Band eingekoppelt wird. Die Strahlung verteilt sich dann im Band und wird aus dem Band radial nach innen in Richtung auf die Sklera abgestrahlt (wie oben beschrieben, bevorzugt homogen). Hierzu kann die Strahlung an einer Umfangsstelle des Bandes eingekoppelt werden und das Band ist so gestaltet, dass sich die Strahlung dann im Band über das gesamte Band, also insbesondere auch in Umfangsrichtung, verteilt und dabei in den einzelnen Lichtleitfasern abgelenkt, zum Beispiel gestreut, wird, sodass sie in Richtung auf die Sklera aus dem Band abgestrahlt wird. Für die genannte Ablenkung (Streuung) können in den Lichtleiterfasern Streukörper eingebettet sein, an denen die Strahlung aus den Lichtleitern herausgekoppelt wird. Dabei kann die Verteilung der Streukörper im Lichtleiter inhomogen sein, derart, dass die oben beschriebene Homogenität der Strahlung erreicht wird. Zum Beispiel kann an der Einkoppelstelle der Strahlung in die Lichtleiterfasern die Dichte der Streukörper geringer sein als an anderen Stellen der Lichtleiterfasern und die Streukörperdichte kann mit zunehmendem Abstand von der Einkoppelstelle anwachsen.

Gemäß einer anderen Ausführungsform ist es auch möglich, im Band selbst winzige lichtemittierende Dioden anzuordnen, derart, dass die oben beschriebene Abstrahlung in die Sklera erreicht wird. Es ist auch denkbar, die Lichtleiterfasern selbst als sogenannte Faserlaser zu gestalten, die z.B. über ihre Länge radial emittieren, vgl. A.M. Stolyarov et al.: "Microfluidic Directional Emission Control Of An Azimuthally Polarized Radial Fibre Laser", Nat. Photonics, online am 11. März 2012; und L. Hardesty: "Fibre Laser Points To Woven 3-D Displays", Mit. Pressemitteilung vom 12. Februar 2012.

Auch können Lichtleiterfasern des Bandes einen Brechungsindexgradienten derart aufweisen, dass die Lichtemission in der oben beschriebenen Weise erfolgt. Eine variierende Reflexionsbeschreibung der Lichtleiterfasern kann auch für diesen Zweck eingesetzt werden.

Eine bevorzugte Ausgestaltung der Erfindung sieht vor, dass das Band auf seiner von der Sklera abgekehrten Seite verspiegelt ist, sodass die hier einfallende Strahlung nach Reflexion ebenfalls in die Sklera eingeleitet wird. Damit können unerwünschte Wirkungen im umliegenden Gewebe (zum Beispiel Muskeln, Bindehaut etc.) verhindert werden.

Die obigen Maßnahmen zur Auskopplung der Strahlung aus dem Band in Richtung auf die Sklera können wahlweise kombiniert werden.

Gemäß einer bevorzugten Ausgestaltung der Erfindung ist die Länge des Bandes in situ, also bei der Operation am Augapfel, einstellbar. Diese Längeneinstellung des gürtelartigen Bandes erfolgt so, dass das Band für eine Strahlungsabgabe in die Sklera optimal positioniert ist, ohne den Augapfel erheblich einzuschnüren. Das gürtelartige Band wird also satt auf die Sklera aufgelegt oder verläuft unmittelbar benachbart zur Sklera. Dabei können Einlagekörper in Umfangsrichtung unter Abstand zwischen dem Band und der Sklera angeordnet sein. Die Längeneinstellung des gürtelartigen Bandes kann zum Beispiel in der Art eines Klettverschlusses, mit kleinen Häkchen oder mittels einer dünnen, zwei Bandenden umschließenden Manschette erfolgen.

Die oben genannten Merkmale der Erfindung können wahlweise miteinander kombiniert werden. Dies gilt auch für alle in dieser Beschreibung und in den Ansprüchen sowie in den Figuren enthaltenen Merkmale.

Die Wellenlänge der genannten elektromagnetischen Strahlung richtet sich nach den Absorptionsbandes des Photomediators. Bei Bengalrosa liegt die Wellenlänge im grünen, bei Riboflavin im UV-Bereich und im blauen optischen Bereich. Bevorzugt für die hier beschriebene sklerale Anwendung ist die blaue Absorptionsbande.

Gemäß einer Weiterbildung der Erfindung kann auch analog zum oben beschriebenen gürtelförmigen Band ein kleineres Stück, zum Beispiel in runder Haubenform, am hinteren Pol das Auges angelegt werden und damit kann analog dem hier näher beschriebenen gürtelförmigen Band der Polbereich mechanisch stabilisiert ("vernetzt") werden um zum Beispiel das sogenannte Staphylom zu verhindern, welches, ähnlich der oben beschriebenen Achsenmyopie, auf eine Sklera mit zu geringer mechanischer Stabilität beruht. Dabei können für die Haube alle im Zusammenhang mit dem Band genannten Merkmale ebenfalls eingesetzt werden.

Nachfolgend werden Ausführungsbeispiele der Erfindung mit Blick auf die Figuren näher beschrieben.
Fig. 1 zeigt eine Vorrichtung zur Prävention von Achsenmyopie;
Fig. 2 zeigt Teile der Vorrichtung in perspektivischer Ansicht; und
Fig. 3 zeigt eine einzelne Lichtleiterfaser schematisch in stark vergrößerter Darstellung.

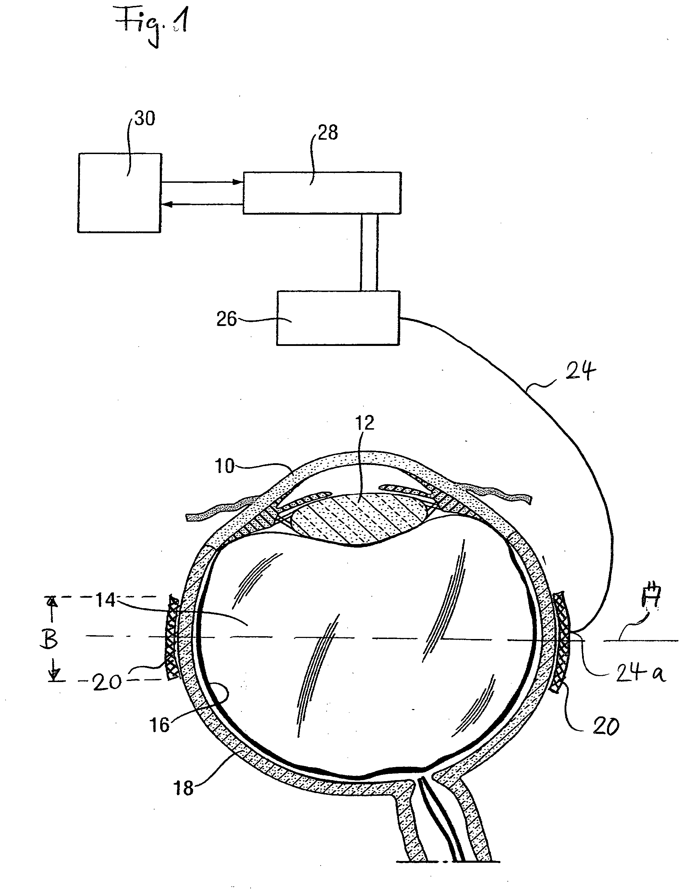

Fig. 1 zeigt schematisch ein Auge mit einer Hornhaut 10, einer Linse 12, einem Glaskörper 14, einer Netzhaut 16 und einer Lederhaut (Sklera) 18. Der Äquator des Auges in schematisch mit "Ä" gekennzeichnet.

Ein Band 20 ist beiderseits des Äquators Ä im Wesentlichen um den gesamten Umfang des Augapfels gelegt. Es wird weiter unten näher beschrieben.

Beim dargestellten Ausführungsbeispiel wird elektromagnetische Strahlung (zum Beispiel im blauen Bereich) in einer Strahlungsquelle 26 erzeugt, zum Beispiel mittels eines Lasers, einer LED etc. Die Strahlungsquelle 26 wird über eine Stromversorgung 28 betrieben, welche ihrerseits von einer Steuerung 30 gesteuert wird. Mit der Steuerung 30 wird insbesondere die Stärke und die Zeitdauer der von der Strahlungsquelle 26 in eine Lichtleitung 24 gegebenen Strahlung eingestellt. Über die Lichtleitung 24 wird die elektromagnetische Strahlung dem Band 20 zugeführt und dort zum Beispiel an der Stelle 24a in das Band 20 eingekoppelt.

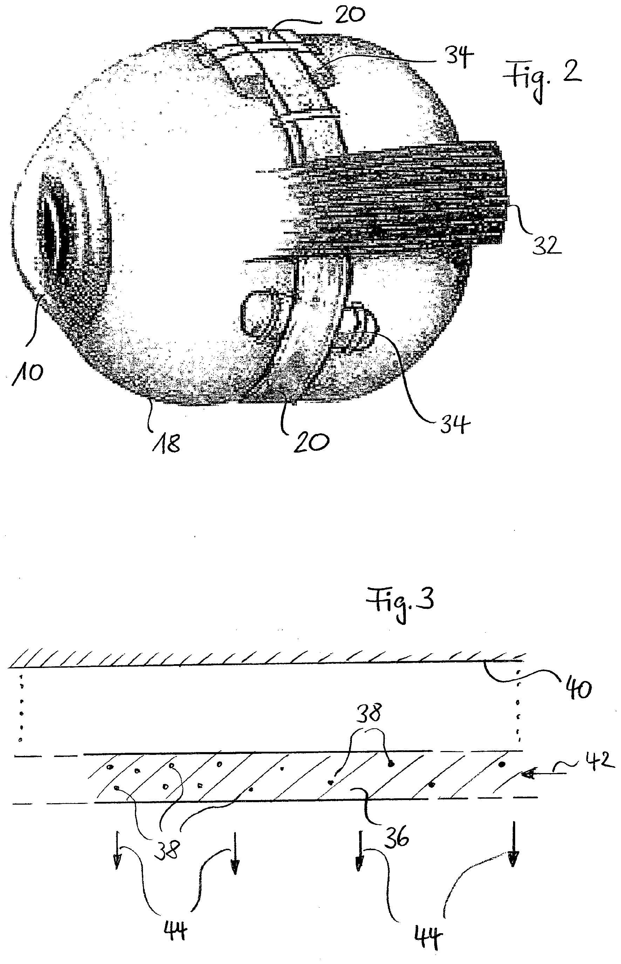

Fig. 2 zeigt den gürtelartigen Einsatz des Bandes 20 am Augapfel. Wie oben beschrieben (Cerclage-Operationstechnik) wird das Band 20 unter die Muskeln gelegt, sodass es unmittelbar den Augapfel umfängt, ohne diesen nennenswert zu komprimieren oder einzuschnüren. Weitere Muskeln sind der Klarheit der Darstellung wegen weggelassen.

Zwischen Band 20 und Augapfel sind beim in Fig. 2 dargestellten Ausführungsbeispiel Einlagen 34 angeordnet.

Das Band 20 besteht aus einem Gewebe aus Lichtleiterfasern, die zwecks Verbesserung der mechanischen Eigenschaften des Bandes, wie zum Beispiel einer gewünschten Weichheit und Elastizität, mehr oder weniger mit Textilfasern gemischt sein können. Das Band 20 hat senkrecht zur Äquatorialebene die oben angegebenen Werte bezüglich der Breite B.

Fig. 3 zeigt in stark vergrößerter Darstellung eine einzelne Lichtleiterfaser 36 aus dem Band 20. Die elektromagnetische Strahlung aus der Strahlungsquelle 26 tritt entsprechend dem Pfeil 42 in die Lichtleiterfaser 36 ein und wird dort an Streukörpern 38 gestreut. Außenseitig des Bandes 20, also an dessen von der Sklera abgekehrter Oberfläche, ist eine reflektierende Fläche 40 vorgesehen, sodass an den Streukörpern 38 radial nach außen gestreute Strahlung zurück in das Band und durch das Band auf die Sklera reflektiert wird. Insgesamt verlässt die elektromagnetische Strahlung das Band 20 in Richtung der Pfeile 44 von Fig. 3 und wird so in die Sklera eingekoppelt, um dort den Photomediator zu aktivieren und so die viskoelastischen Eigenschaften der Sklera zu ändern und damit einer Verlängerung der Augenachse aufgrund einer "kriechenden" Dehnung und Verlängerung der Sklera entgegenzuwirken und damit Achsenmyopie zu verhindern.

Auch die oben genannten Maßnahmen, also ein Brechungsindexgradient und/oder eine Reflexionsbeschichtung der Lichtleiterfasern können wahlweise für die Auskopplung der Strahlung aus dem Band eingesetzt werden.

In Abwandlung des vorstehend beschriebenen Ausführungsbeispieles kann die Strahlungsquelle auch direkt am oder im Band 20 angeordnet werden. Bei dieser Abwandlung wird einer oder mehreren Strahlungsquellen im Band die erforderliche elektrische Energie über eine Leitung analog der Leitung 24 gemäß Fig. 1 zugeführt.

Zur Behandlung eines Staphyloms sieht ein beispielhaftes Verfahren folgendes vor: eine Haube, die eingerichtet ist, auf den hinteren Polbereich des Auges aufgelegt oder benachbart des Polbereichs angeordnet zu werden, und eine oder mehrere Strahlungsquellen zur Erzeugung elektromagnetischer Strahlung, die in der Haube erzeugbar oder in die Haube einkoppelbar ist, um in den Polbereich der Sklera gestrahlt zu werden.

## Patentansprüche

1. Vorrichtung zur Vorbeugung und/oder Behandlung von Achsenmyopie des Auges, aufweisend:
- zumindest ein Band (20), das eingerichtet ist, von außen auf die Sklera (18) aufgelegt oder benachbart der Sklera (18) angeordnet zu werden,
- eine oder mehrere Strahlungsquellen (26) zur Erzeugung von elektromagnetischer Strahlung, die im Band (20) erzeugt wird oder in das Band (20) einkoppelbar ist, um in die Sklera (18) gestrahlt zu werden,
wobei das Band (20) eingerichtet ist, äquatorial um das Auge gelegt zu werden
**dadurch gekennzeichnet, dass** das Band (20) aus einem Gewebe besteht, das entweder aus Lichtleitfasern (36) oder aus einer Mischung von Textilfasern und Lichtleitfasern (36) besteht.

2. Vorrichtung nach Anspruch 1, **dadurch gekennzeichnet, dass** das Band (20) eine Breite (B) von 3 bis 12 mm hat.

3. Vorrichtung nach Anspruch 1, **dadurch gekennzeichnet, dass** das Band (20) eine Breite (B) von 4 bis 10 mm hat.

4. Vorrichtung nach Anspruch 1, **dadurch gekennzeichnet, dass** das Band (20) eine Breite (B) von 5 bis 8 mm hat.

5. Vorrichtung nach einem oder mehreren der vorhergehenden Ansprüche, **dadurch gekennzeichnet, dass** das Band (20) dazu eingerichtet ist, elektromagnetische Strahlung homogen in Richtung auf die Sklera (18) abzustrahlen.

6. Vorrichtung nach einem oder mehreren der vorhergehenden Ansprüche, **dadurch gekennzeichnet, dass** das Band (20) auf seiner von der Sklera (18) abgekehrten Seite zumindest teilweise verspiegelt ist.

7. Vorrichtung nach einem oder mehreren der vorhergehenden Ansprüche, **dadurch gekennzeichnet, dass** die Strahlungsquelle oder die Strahlungsquellen am oder in dem Band (20) angeordnet sind.

8. Vorrichtung nach einem oder mehreren der Ansprüche 1 bis 6, **dadurch gekennzeichnet, dass** die Strahlungsquelle (26) oder die Strahlungsquellen extern des Bandes (20) angeordnet sind und die Strahlung über einen oder mehrere Lichtleiter (24) in das Band (20) eingekoppelt wird.

9. Vorrichtung nach einem oder mehreren der vorhergehenden Ansprüche, **dadurch gekennzeichnet, dass** die Lichtleiterfasern (36) Streukörper (28) aufweisen zur Auskoppelung von Strahlung quer zur Achse der Lichtleiterfasern (36).

10. Vorrichtung nach einem oder mehreren der vorhergehenden Ansprüche, **dadurch gekennzeichnet, dass** die Länge des Bandes (20) in situ einstellbar ist.

11. Vorrichtung nach einem oder mehreren der vorhergehenden Ansprüche, mit mehreren Einlagen (34), die eingerichtet sind, zwischen dem Band (20) und der Sklera (18) angeordnet zu werden.

12. Vorrichtung nach einem oder mehreren der vorhergehenden Ansprüche, **dadurch gekennzeichnet, dass** die Wellenlänge der Strahlung im Bereich von 300 bis 800 nm liegt.

13. Vorrichtung nach einem oder mehreren der vorhergehenden Ansprüche, **dadurch gekennzeichnet, dass** die Wellenlänge der Strahlung im Bereich von 450 bis 470 nm oder 500 bis 520 nm liegt.

14. Vorrichtung zur Vorbeugung und/oder Behandlung des Staphyloms, aufweisend:
- eine Haube, die eingerichtet ist, auf den hinteren Polbereich des Auges aufgelegt oder benachbart des Polbereichs angeordnet zu werden, und
- eine oder mehrere Strahlungsquellen (26) zur Erzeugung elektromagnetischer Strahlung, die in der Haube erzeugbar oder in die Haube einkoppelbar ist, um in den Polbereich der Sklera (18) gestrahlt zu werden,
**dadurch gekennzeichnet, dass** die Haube aus einem Gewebe besteht, das entweder aus Lichtleitfasern (36) oder aus einer Mischung von Textilfasern und Lichtleitfasern (36) besteht.

## Claims

1. A device for preventing and/or treating axial myopia of the eye, having:
- at least one band (20) that is configured for being placed on the sclera (18) from the outside, or adjacent to the sclera (18),
- one or more radiation sources (26) for generating electromagnetic radiation that is generated in the band (20) or coupleable into the band (20) in order to be radiated into the sclera (18),
wherein the band (20) is configured for being laid equatorially around the eye,
**characterized in that** the band (20) is made of a fabric composed of either optical fibers (36) or a mixture of textile fibers and optical fibers (36).

2. The device according to Claim 1, **characterized in that** the band (20) has a width (B) of 3 to 12 mm.

3. The device according to Claim 1, **characterized in that** the band (20) has a width (B) of 4 to 10 mm.

4. The device according to Claim 1, **characterized in that** the band (20) has a width (B) of 5 to 8 mm.

5. The device according to one or more of the preceding claims, **characterized in that** the band (20) is configured for homogeneously irradiating electromagnetic radiation toward the sclera (18).

6. The device according to one or more of the preceding claims, **characterized in that** the band (20) is at least partially metallized on its side facing away from the sclera (18).

7. The device according to one or more of the preceding claims, **characterized in that** the radiation source or the radiation sources is/are situated on or in the band (20).

8. The device according to one or more of Claims 1 to 6, **characterized in that** the radiation source (26) or the radiation sources is/are situated external to the band (20), and the radiation is coupled into the band (20) via one or more waveguides (24).

9. The device according to one or more of the preceding claims, **characterized in that** the optical fibers (36) have scattering bodies (28) for decoupling radiation transversely with respect to the axis of the optical fibers (36).

10. The device according to one or more of the preceding claims, **characterized in that** the length of the band (20) is adjustable in situ.

11. The device according to one or more of the preceding claims, having multiple inserts (34) that are configured for being situated between the band (20) and the sclera (18).

12. The device according to one or more of the preceding claims, **characterized in that** the wavelength of the radiation is in the range of 300 to 800 nm.

13. The device according to one or more of the preceding claims, **characterized in that** the wavelength of the radiation is in the range of 450 to 470 nm or 500 to 520 nm.

14. A device for preventing and/or treating staphyloma, having:
- a hood that is configured for being placed on the rear pole area of the eye or adjacent to the pole area, and
- one or more radiation sources (26) for generating electromagnetic radiation that is generatable in the hood or coupleable into the hood in order to be radiated into the pole area of the sclera (18),
**characterized in that** the hood is made of a fabric composed of either optical fibers (36) or a mixture of textile fibers and optical fibers (36).

## Revendications

1. Dispositif de prévention et/ou de traitement de la myopie axiale de l'oeil, comprenant :
- au moins une bande (20) agencée pour être appliquée à la sclérotique (18) depuis l'extérieur ou pour être disposée à proximité de la sclérotique (18),
- une ou plusieurs sources de rayonnement (26) pour générer un rayonnement électromagnétique généré dans la bande (20) ou pouvant être injecté dans la bande (20) afin d'être émis dans la sclérotique (18),
la bande (20) étant agencée pour être placée autour de l'oeil de manière équatoriale, **caractérisé en ce que** la bande (20) se compose d'un tissu composé soit de fibres optiques (36), soit d'un mélange de fibres textiles et de fibres optiques (36).

2. Dispositif selon la revendication 1, **caractérisé en ce que** la bande (20) présente une largeur (B) comprise entre 3 et 12 mm.

3. Dispositif selon la revendication 1, **caractérisé en ce que** la bande (20) présente une largeur (B) comprise entre 4 et 10 mm.

4. Dispositif selon la revendication 1, **caractérisé en ce que** la bande (20) présente une largeur (B) comprise entre 5 et 8 mm.

5. Dispositif selon une ou plusieurs des revendications précédentes, **caractérisé en ce que** la bande (20) est agencée pour émettre un rayonnement électromagnétique homogène en direction de la sclérotique (18).

6. Dispositif selon une ou plusieurs des revendications précédentes, **caractérisé en ce que** la bande (20) est au moins partiellement revêtue d'une couche réfléchissante sur un côté détourné de la sclérotique (18).

7. Dispositif selon une ou plusieurs des revendications précédentes, **caractérisé en ce que** la source de rayonnement ou les sources de rayonnement est/sont disposée(s) sur ou dans la bande (20).

8. Dispositif selon une ou plusieurs des revendications 1 à 6, **caractérisé en ce que** la source de rayonnement (26) ou les sources de rayonnement est/sont disposée(s) à l'extérieur de la bande (20) et le rayonnement est injecté dans la bande (20) par l'intermédiaire d'un ou de plusieurs guide(s) d'ondes (24).

9. Dispositif selon une ou plusieurs des revendications précédentes, **caractérisé en ce que** les fibres optiques (36) disposent de diffuseurs (28) pour déclencher un rayonnement transversalement à l'axe des fibres optiques (36).

10. Dispositif selon une ou plusieurs des revendications précédentes, **caractérisé en ce que** la longueur de la bande (20) peut être réglée in situ.

11. Dispositif selon une ou plusieurs des revendications précédentes, comprenant plusieurs inserts (34) agencés pour être disposés entre la bande (20) et la sclérotique (18).

12. Dispositif selon une ou plusieurs des revendications précédentes, **caractérisé en ce que** la longueur d'onde du rayonnement se situe dans la plage allant de 300 à 800 nm.

13. Dispositif selon une ou plusieurs des revendications précédentes, **caractérisé en ce que** la longueur d'onde du rayonnement se situe dans la plage allant de 450 à 470 nm ou de 500 à 520 nm.

14. Dispositif de prévention et/ou de traitement du staphylome, comprenant :
- une calotte agencée pour être posée sur la zone polaire arrière de l'oeil ou à proximité de la zone polaire, et
- une ou plusieurs sources de rayonnement (26) pour générer un rayonnement électromagnétique pouvant être généré dans la calotte ou pouvant être injecté dans la calotte afin d'être émis dans la zone polaire de la sclérotique (18),
**caractérisé en ce que** la calotte se compose d'un tissu composé soit de fibres optiques (36) soit d'un mélange de fibres textiles et de fibres optiques (36).
